(19) Europäisches Patentamt
European Patent Office
Office européen des brevets

(11) Publication number: 0 313 858
A2

# (12) EUROPEAN PATENT APPLICATION

(21) Application number: 88116201.0

(22) Date of filing: 30.09.88

(51) Int. Cl.4: C12Q 1/00 , //C12Q1/40, G01N33/52,G01N33/543

The title of the invention has been amended (Guidelines for Examination in the EPO, A-III, 7.3).

(30) Priority: 01.10.87 JP 248748/87

(43) Date of publication of application: 03.05.89 Bulletin 89/18

(84) Designated Contracting States: DE FR GB

(71) Applicant: FUJI PHOTO FILM CO., LTD.
210 Nakanuma Minami Ashigara-shi
Kanagawa 250-01(JP)

(72) Inventor: Shinoki, Hiroshi c/o Fuji Photo Film Co., Ltd.
11-46, Senzui, 3-chome
Asaka-shi Saitama(JP)
Inventor: Sudo, Yukio c/o Fuji Photo Film Co., Ltd.
11-46, Senzui, 3-chome
Asaka-shi Saitama(JP)
Inventor: Kageyama, Shigeki c/o Fuji Photo Film Co., Ltd.
11-46, Senzui, 3-chome
Asaka-shi Saitama(JP)
Inventor: Ono, Mitsunori
c/o Fuji Photo Film Co., Ltd. 210, Nakanuma
Minami Ashigara-shi Kanagawa(JP)

(74) Representative: Patentanwälte Grünecker, Kinkeldey, Stockmair & Partner
Maximilianstrasse 58
D-8000 München 22(DE)

## (54) Analytical element.

(57) A multi-layer dry-type chemical analyzing element capable of analysis of a particular component in an aqueous liquid sample, which comprises at least two water-permeable layers and which is wherein one of the at least two water-permeable layers is a substrate layer containing a substrate of an enzyme and the other water-permeable layer is a color reaction layer, the substrate is chemically bound with a dye moiety with a temporarily hypsochromically shifted spectral absorption maximum to form a non-diffusible substance which reacts with a substance having an enzyme activity to form a diffusible substance containing a dye moiety with a temporarily hypsochromically shifted spectral absorption in the molecule, and the color reaction layer contains a reactive substance which reacts with the diffusible substance to thereby lengthen the wavelength of the maximum spectral absorption of the temporarily shifted dye moiety.

Fig. 1

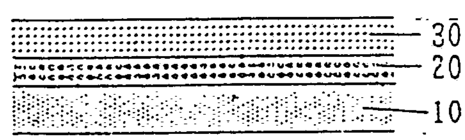


EP 0 313 858 A2

# ANALYZING ELEMENT

## FIELD OF THE INVENTION

The present invention relates to an analyzing element useful for measurement of enzyme activity in an aqueous liquid, especially humor, as well as for immunological assays by employing enzyme labeling.

## BACKGROUND OF THE INVENTION

Analysis of constitutional components in a humor of a living body, such as blood or urine, as well as drug components therein is extremely helpful for pathologic diagnosis or for judgement of therapeutic progress and therefore has an important role in the field of clinical tests. An analytical method of quantitatively determining the biochemical substances contained in humor or the like with a dry-type analyzing element is known. In accordance with a dry-type analyzing element, in general, the amount of the reaction product formed by reaction of the component in a test sample with a reagent contained in the analyzing element or the amount of a non-reacted component is measured optically, for example, by spectrophotometry on the basis of the coloration, discoloration, fluorescence or light-emission from the reaction product or the non-reacted component, whereby the amount of the component to be detected is determined. By the use of the dry-type analyzing element, therefore, it is possible to analyze a particular component, for example, a biochemically active substance in a humor, simply, rapidly and highly accurately.

Activities of various enzymes are especially important for diagnosis and therapy of diseases. A dry-type analyzing element is also useful for measurement of enzyme activity, as disclosed, for example, in Analytical Chemistry, Vol.55, No.4, pages 504A and 506A (April, 1983); and JP-A-55-124499 (U.S. Patent 4,269,938), 58-155100 (U.S. Patent 4,450,232), 60-43400, 57-40649 (British Patent 2,085,159), 57-144996 (U.S. Patent 4,503,145), 57-208998, 59-30063, 59-42897, 60-95349, 61-110058 (European Patent Application 0182179A). (The term "JP-A" as used herein means an "unexamined published Japanese patent application".)

Further, a dry-type analyzing element is useful also for immunological assays. For example, a dry-type analyzing element of the kind described in JP-A-49-53888 (U.S. Patent 3,992,158), 59-77356, 59-102388, U.S. Patent 4,459,358, etc. are known. The antigen-antibody reaction used in an immunological assay is a reaction where an antigen or antibody specifically reacts with only the corresponding antibody or antigen, respectively, and thereby is bound thereto, and such is being widely utilized for diagnosis of autoimmune diseases or for detection of minor constituents in the living body. Enzyme immunoassay (EIA) is known as a method of immunological assay having a relatively high detection sensitivity. One typical method is illustrated in JP-B-53-27763 (U.S. Patents 3,817,837 and 3,852,157) (the term "JP-B" as used herein means an "examined Japanese patent publication"), in which an enzyme is bound to an antibody or a derivative thereof to form an enzyme-labeled antibody and the antigen in the test liquid is assayed by the measurement of the effect of the antigen to the enzyme activity.

The dry-type analyzing element described in JP-A-53-131089 (U.S. Patent 4,144,306) is a multi-layer analyzing element which comprises a reagent layer containing a non-diffusible substance formed by previously binding a chromophore-containing group having a detectable spectral absorption, such as that of a dye, to a substrate of an enzyme, such as a starch, and a detecting layer which accepts the diffusible reaction product formed by the action of the substance to be detected, such as an enzyme. The optical density of the absorption of the colored chemical group, such as that of a dye, of the resulting reaction product given in accordance with the amount of the reaction product as accepted by the detecting display layer of the analyzing element, is measured to thereby determine the amount of the substance to be detected.

In this analyzing element, however, since a chromophoric group having a detectable spectral absorption is previously attached to a non-diffusible substrate, the non-reacted substance and the reaction product have to be distinguished from each other in the optical measurement. Accordingly, JP-A-53-131089 (U.S. Patent 4,144,306) has proposed one embodiment for the optical discrimination in which a light-shielding layer containing titanium oxide particles or the like is provided between the reagent layer containing the

non-diffusible non-reacted substrate and the detecting display layer which accepts the diffusible reaction product. However, when the analyzing element having such a layer constitution is used for quantitative analysis, the time required for the diffusible reaction product formed in the reagent layer to sufficiently diffuse to the detecting layer through the light-shielding layer must be taken into consideration, and such time apparently deteriorates the advantage in the dry-type chemical analysis characterized by a rapid quantitative determination capacity.

In order to accelerate the diffusion of the reaction product, it has been tried to introduce two or more diffusible groups, such as a carboxyl group or a sulfo group, into the dye moiety (the chemical moiety having a chromophoric group with a detectable spectral absorption) in the diffusible compound. However, the positions into which the substituents can be introduced are limited, and the introduction of such substituents lowers the molecular extinction coefficient of the reaction product having the dye moiety which is crucial the sensitivity of the analyzing element.

## SUMMARY OF THE INVENTION

Accordingly, the object of the present invention is to overcome the above-mentioned problems and to provide an improved dry-type multilayer chemical analyzing element in which a non-diffusible substance capable of releasing a detectable diffusible compound by enzymatic reaction is utilized for determination of enzymatic activity of the enzyme of an enzyme-labeled body whereby a rapid analysis is possible and high analytical sensitivity and analytical accuracy can be obtained.

More specifically, the present invention relates to an improved dry-type analyzing element based on the above-mentioned principle, which is characterized in that no light-shielding layer is required between the layer containing the non-diffusible substance and the layer accepting the diffusible compound and accordingly the diffusion of the diffusible compound formed is accele rated. Furthermore, the non-diffusible substance releases a diffusible compound which can easily diffuse in accordance with the activity or amount of the substance to be detected, and can form a substance having a spectral wavelength which can be easily distinguished from those of the non-diffusible substance, and a high extinction coefficient at the detection wavelength.

The object of the present invention can be attained by a multi-layer dry-type chemical analyzing element comprising at least two water-permeable layers and which is capable of quantitative determination of an enzyme contained in an aqueous liquid sample or of a substance capable of forming or decreasing an enzyme-carrying substance in accordance with the content of the substance in an aqueous liquid sample, wherein one of the at least two water-permeable layers is a substrate layer containing a substrate of the enzyme and the other water-permeable layer is a color reaction layer, the substrate is chemically bound with a dye moiety with a temporarily hypsochromically-shifted spectral absorption maximum to form a non-diffusible substance which reacts with a substance having an enzyme activity to form a diffusible compound having a dye moiety with a temporarily shifted spectral absorption maximum in the molecule, and the color reaction layer contains a reactive substance which reacts with the diffusible compound to thereby lengthen the wavelength of the maximum spectral absorption of the temporarily hypsochromically absorption-shifted dye moiety.

## BRIEF DESCRIPTION OF THE DRAWINGS

Fig. 1, Fig. 2 and Fig. 3 each shows a sectional view of an embodiment of the analyzing element of the present invention.

Fig. 4 is a graph to show optical density variations of colorimetric devices.

## DETAILED DESCRIPTION OF THE INVENTION

The constitution and composition of the multi-layer chemical analyzing element of the present invention is explained hereafter. Reference is made to Fig. 1 which shows a sectional view of one embodiment of the present invention. The multi-layer chemical analyzing element of Fig. 1 is composed of a color reaction

layer (20) and a substrate layer (30) laminated on a light-transmissible and water-impermeable support (10) in order.

The substrate layer (30) contains a non-diffusible substance derived by binding a substrate with a dye moiety with a temporarily shortened maximum absorption wavelength. The non-diffusible substance forms a diffusible compound capable of diffusing through a water-permeable medium in the presence of water by the action of the substance to be tested which is an enzyme, or by the action of an enzyme-carrying substance (enzyme-bound substance) to be formed or decreased in accordance with the amount of the substance to be tested. The diffusible compound contains a dye moiety with a temporarily shortened maximum absorption wavelength released from the non-diffusible substance.

The color reaction layer (20) accepts the diffusible compound having a dye moiety with a temporarily shortened maximum absorption wavelength and contains a reagent which reacts with the dye moiety of the diffusible compound so as to change the shortened maximum absorption wavelength to a longer wavelength to render the diffusible compoun colored.

The substrate layer and the color reaction layer each may be a non-porous layer comprising a hydrophilic binder. When both layers comprise a hydrophilic binder, the hydrophilic binder for the two layers may be the same or different. In accordance with each of the substances to be tested, the non-diffusible substance, the diffusible compound and the color restoration reagent and the combination thereof, a suitable hydrophilic binder can be selected for each layer.

The non-diffusible substance and the color restoration reagent are preferably separated from each other. For example, a separating layer (40) so as to prevent contact between the non-diffusible substance and the color restoration reagent may be provided between the substrate layer (30) and the color reaction layer (20), as shown in Fig. 2. Fig. 3 shows a sectional view of another preferred embodiment of the analyzing element of the present invention, which is composed of a color reaction layer (20), a separating layer (40), a substrate layer (30) and a spreading layer (50) laminated on a light-transmissible and water-impermeable support (10) in this order.

Referring to the drawings, the function of the analyzing element (processes for analysis with the element) of the present invention will be explained. When one drop of an aqueous liquid sample containing a substance to be tested is applied to the spreading layer (50), the sample is spread nearly uniformly on the spreading layer and permeates into the substrate layer (30). A diffusible compound is released from the non-diffusible substance in the substrate layer (30) by the action of the substance to be tested (e.g., enzyme) as contained in the sample or of an enzyme-carrying substance to be formed or decomposed in accordance with the amount of the substance to be tested in the sample, and the thus formed diffusible compound then diffuses from the substrate layer (30) to the color reaction layer (20) through the separating layer (40). Since the diffusible compound which is the reaction product has a non-diffusible substance-derived and temporarily absorption-shifted dye moiety bound thereto, this immediately reacts with the color restoration reagent, when the color reaction layer (20) is reached, so as to be converted to a longer wavelength color. The shifting width in the wavelength by the reaction preferably falls within the range of from 30 nm to 150 nm, and more preferably from 50 nm to 150 nm. The details of the technique of using a temporarily absorption-shifted dye and then rapidly color restoring by lengthening the wavelength is described hereunder.

The aforesaid substance to be tested may be either an enzyme which directly reacts with the non-diffusible substance or an enzyme-carrying substance which is formed or decomposed as a result of a specific protein-bonding reaction with a substance to be tested (typically immunological antigen-antibody reactions), for example, an enzyme-labeled antigen or antibody which may indirectly react with the substrate by the enzyme reaction thereof. A protein so labeled that the action to the substrate is modified as a result of the competing protein-binding reaction between the substance to be tested (test substance) and the analogues of the test substance can also be used. The enzyme-carrying substance may be previously formed in an aqueous medium outside the analyzing element, or alternatively, this may be formed in a suitable layer in the multi-layer chemical analyzing element of the present invention. A reaction layer for forming an enzyme or an enzyme-carrying substance from the substance to be tested may additionally be provided in the analyzing element of the present invention. The layer may be integrated with the analyzing element or may be temporarily contacted with the element.

The non-diffusible substance remains in the substrate layer (30), and only the diffusible compound resulting from the enzyme reaction diffuses into the color reaction layer (20) and color is formed therein. Since the change in the amount of the diffusible compound depends upon the enzyme activity with the non-diffusible substance, the compound is rendered colored as a result, in accordance with the enzyme activity so that the enzyme activity can be determined by measurement of the amount of the colored compound. When the substance to be tested is not an enzyme but a substance which forms or decomposed an

enzyme-carrying substance in accodance with the amount of the substance to be tested, the enzyme activity of the enzyme-carrying substance can be determined by the process of the present invention and the amount of the substance to be tested can thereby be determined.

For measurement of the amount of the colored substance, a method of optical measurement in trms of the transmittance or reflection in the absorption wavelength range of the colored substance is suitable, but in some cases the amount can be judged visually with the naked eye in accordance with the object and the accuracy required.

Next, the principle of the reaction between the temporarily absorption-shifted dye moiety-having non-diffusible substance contained in the substrate layer and the color restoration reagent cotained in the color reaction layer in the present invention are explained. The reaction is based upon a phenomenon of a so-called halochromism, that is, the absorption maximum value of wavelength of the dye of which the auxochrome is the dissociated state changes when the auxochrome converts into the non-dissociated state.

Specific examples of the dyes which exhibits such halochromism are described in detail, for example, in J. Fabian & H. Hartmann, Light Absorption of Organic Colorants (published by Springer-Verlag). In the present invention, the maximum absorption wavelength of the dye moiety to be used for the detection is preferably from 450 nm to 650 nm, more preferably from 500 nm to 650 nm. Examples of dyes used in the present invention include an azo dye, an oxonol dye, a xantene dye, and a azomethine dye.

The auxochrome in the dye moiety to be used in the present invention must be able to dissociate, for example, under neutral aqueous conditions, after the color reaction. Accordingly, the auxochrome desirably has a pKa value of 6.0 or less. The limitation, however, does not apply to the case where the color reaction layer or the detecting layer contains a mordant. For the purpose of fixing the dye moiety auxochrome of the non-diffusible substance in the non-dissociated state (for the purpose of protecting the reactive group from the viewpoint of organic chemistry), any group may be used which can be released by attack of a nucleophilic reagent. The group can be selected from those described, for example, in J.F.W. Macormy, Protective Group in Organic Chemistry (published by Plenum Press Co., 1973) and T.W. Green, Protective Group in Organic Synthesis (published by Willey Interscience Co., 1981).

Examples for such group include an aliphatic or aromatic acyl group, an alkyl or aryl sulfonyl group and $PO(OR)_2$- group (wherein R represents H, an alkyl group or an aryl group).

As the compound to be used for the purpose of dissociating the non-dissociated dye moiety (i.e., a dye moiety having a protected auxochrome) in the diffusible compound, any compound having a nucleophilic group can be used. For example, there may be mentioned primary amines, secondary amines, thiols, alkoxides and the like compounds as well as so-called "$\alpha$ effect-having" nucleophilic reagents having a higher nucleophilic force than that to be expected from a general pKa value, such as hydroxamic acids, oximes, hydrazines and hydroxylamines. The "$\alpha$ effect" is described in detail in G. Cropmann, Chemical Activity and Reaction Paths (published by Willey Interscience Co., 1974), pages 203 to 221. The nucleophilic reagents can be used directly as such or can be used in the polymerized form.

In order to make the nucleophilic reagent non-diffusible, the reagent may be previously dissolved in a hydrophobic oily substance or locally incorporated into grains and then dispersed in a hydrophilic binder.

A typical substrate which is used for preparation of the non-diffusible substance is a naturaly occurring polymeric polysaccharide.

For introduction of a protected auxochrome-containing dye moiety into the non-diffusible substrate in the present invention, a conventional technique for "reactive dyes" which is well known in the dye industry can be employed. In the dye industry, in addition to physical adsorption of dye compounds to natural fibers of a wide variety, a chemical bond is formed between the fibers and the dye molecule to attain a better dyed state. The dyes suitable for such chemical bond formation are reactive dyes described in detail in K. Venkatarman, The Chemistry of Synthetic Dyes, Vol. VI (published by Academic Press Co., 1972). In particular, "linking groups" having the function of binding molecules of polysaccharides which are natural polymers, such as cellulose or starch, as well as protein fibers, such as wool or silk, to dye molecules are described in greater detail in the literature, and the synthesis of the non-diffusible substance for use in the present invention can be performed by reference to these techniques. Since the non-diffusible substance having protected dye moiety is required to become a substance having a lower molecular weight, by enzyme reaction, for example, so as to diffuse from the substrate layer to the color reaction layer through a hydrophilic medium, in the element of the present invention, the non-diffusible substance is desired to have a soluble group, for example, a sulfo group, a carboxyl group, a hydroxyl group or a quaternary ammonium group. The soluble group may be introduced either in a part of the linking group or in the protective group of the auxochrome.

The typical example for reactions which proceed in the analyzing element of the present invention upon testing a liquid sample are schematically shown below.

[succharide moiety]$_n$—[dye moiety-protected auxochrome]$_m$
- non-diffusible substance

↓ enzyme

[succharide moiety]$_{n'}$—[dye moiety-protected auxochrome]$_{m'}$
diffusive compound having a lower molecular weight, i.e., $m \gg m'$, $n \gg n'$

↓ a nucleophilic reagent

[succharide moiety]$_{n'}$—[dye moiety-non-protected auxochrome]$_{m'}$

Specific examples of protected auxchrome-having dye moieties for use in the present invention are set forth below, which, however, are not intended to restrict the scope of the present invention.

D-1

After Dissociation: $\lambda_{max}$ 520 nm ($H_2O$)

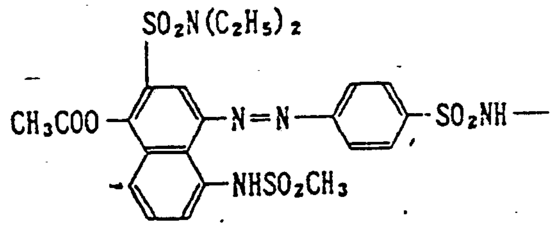

D-2

After Dissociation: $\lambda_{max}$ 460 nm ($H_2O$)

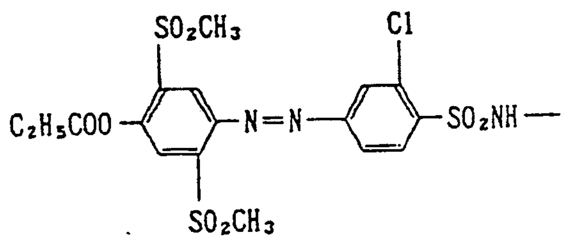

D-3

After Dissociation: $\lambda_{max}$ 640 nm ($H_2O$)

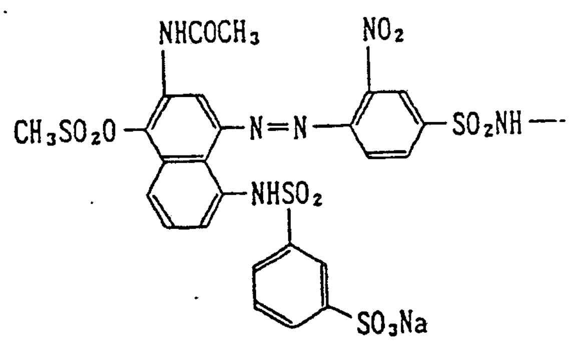

D-4 After Dissociation: $\lambda_{max}$ 520 nm ($H_2O$)

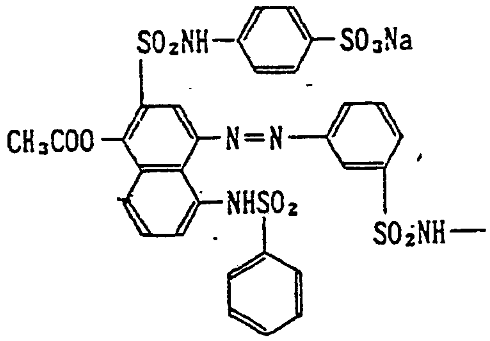

D-5 After Dissociation: $\lambda_{max}$ 550 nm ($H_2O$)

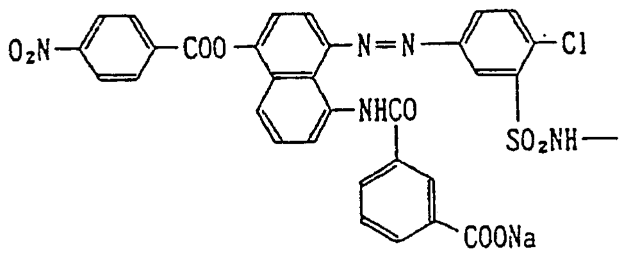

D-6 After Dissociation: $\lambda_{max}$ 505 nm ($H_2O$)

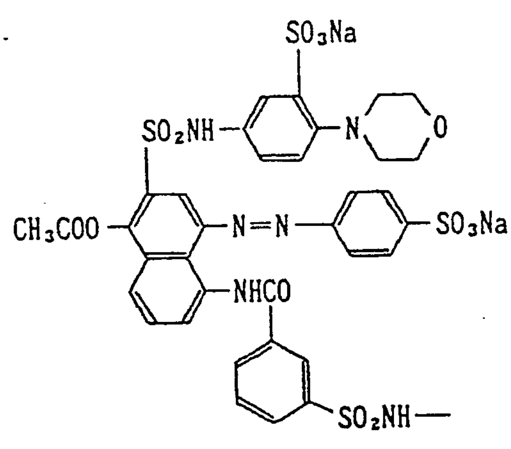

D-7 After Dissociation: $\lambda_{max}$ 530 nm ($H_2O$)

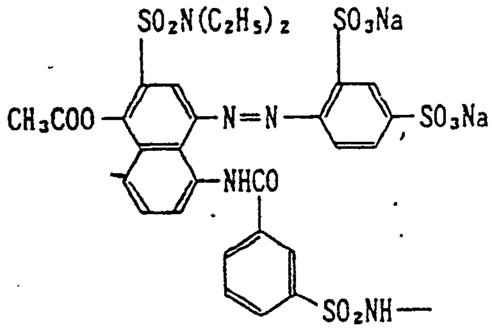

D-8 After Dissociation: $\lambda_{max}$ 620 nm ($H_2O$)

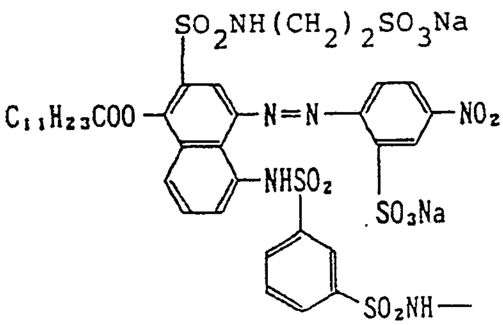

D-9 After Dissociation: $\lambda_{max}$ 517 nm ($H_2O$)

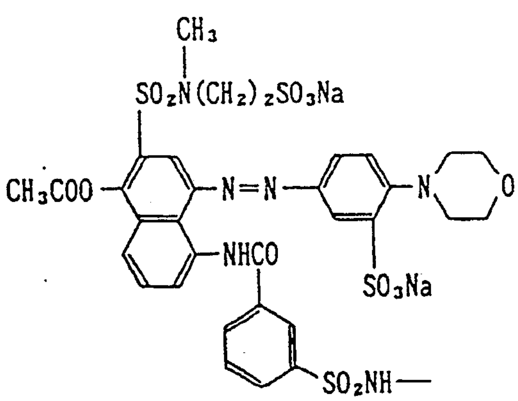

D-10 After Dissociation: $\lambda_{max}$ 510 nm ($H_2O$)

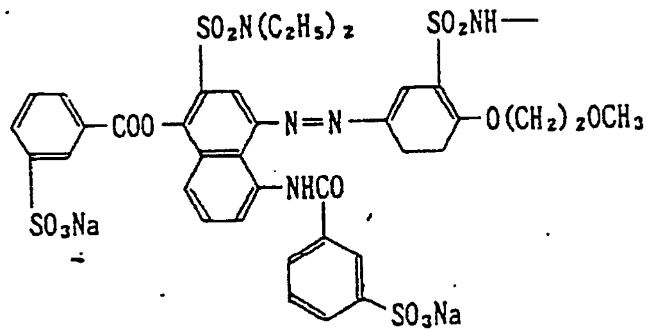

D-11 After Dissociation: $\lambda_{max}$ 620 nm ($H_2O$)

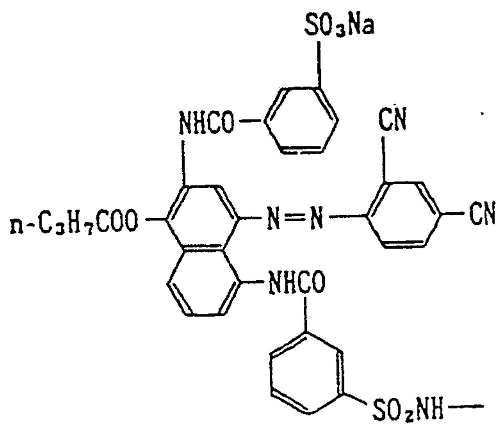

D-12 After Dissociation: $\lambda_{max}$ 630 nm ($H_2O$)

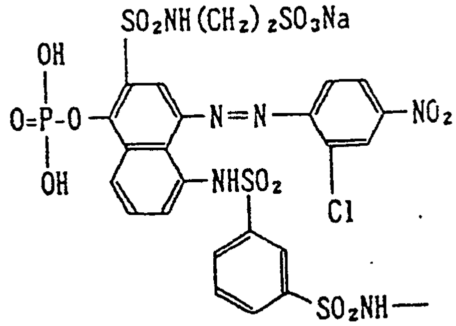

D−13 After Dissociation: $\lambda_{max}$ 515 nm ($H_2O$)

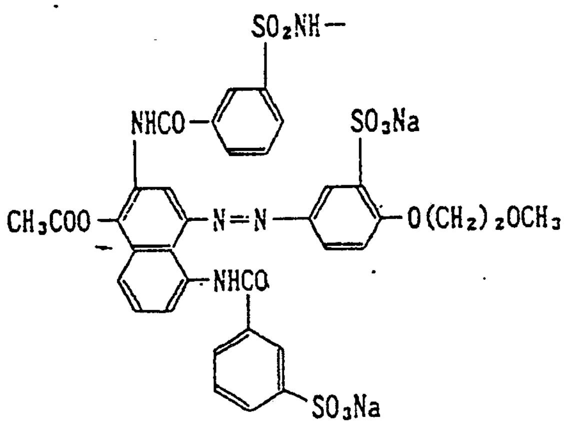

D−14 After Dissociation: $\lambda_{max}$ 505 nm ($H_2O$)

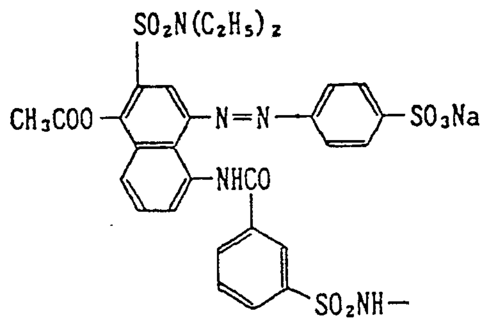

D−15 After Dissociation: $\lambda_{max}$ 650 nm ($H_2O$)

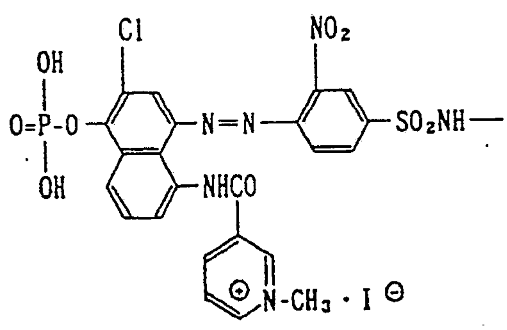

D-16 After Dissociation: $\lambda_{max}$ 550 nm ($H_2O$)

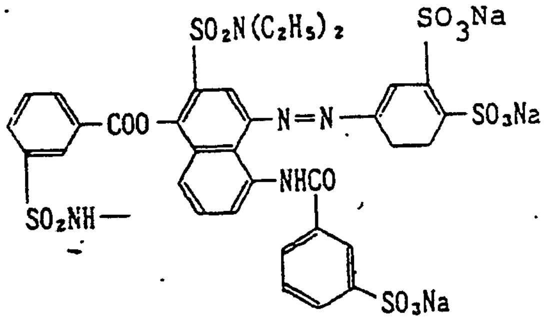

D-17 After Dissociation: $\lambda_{max}$ 610 nm ($H_2O$)

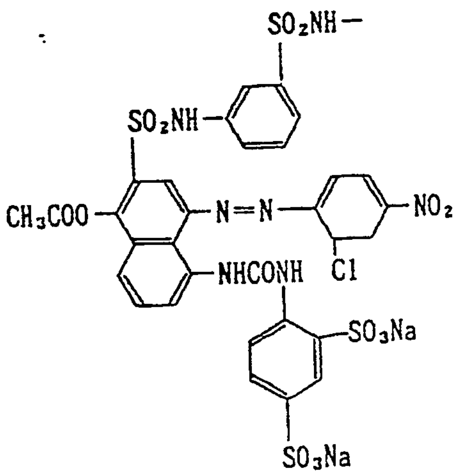

D-18 After Dissociation: $\lambda_{max}$ 520 nm ($H_2O$)

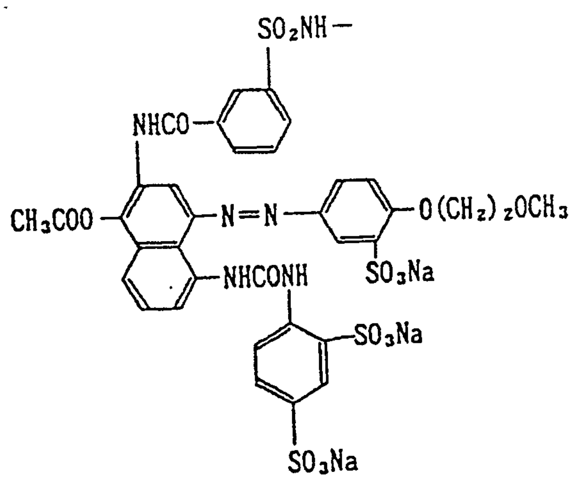

D-19 After Dissociation: $\lambda_{max}$ 630 nm ($H_2O$)

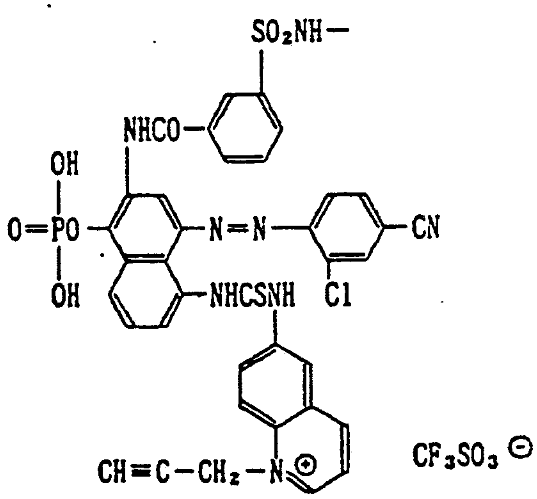

One dye moiety is introduced, for example, to a polysaccharide composed of glucose units, preferably per 5 to 500, more preferably 20 to 200 of glucose units.

The amount of the non-diffusible substance in the analyzing element is preferably from 0.1 to 50 g/$m^2$, more preferably, from 2 to 20 g/$m^2$.

Next, one embodiment of a reaction scheme for producing the temporarily short-shifted dye moiety-containing non-diffusible substance for use in the present invention is illustrated hereunder.

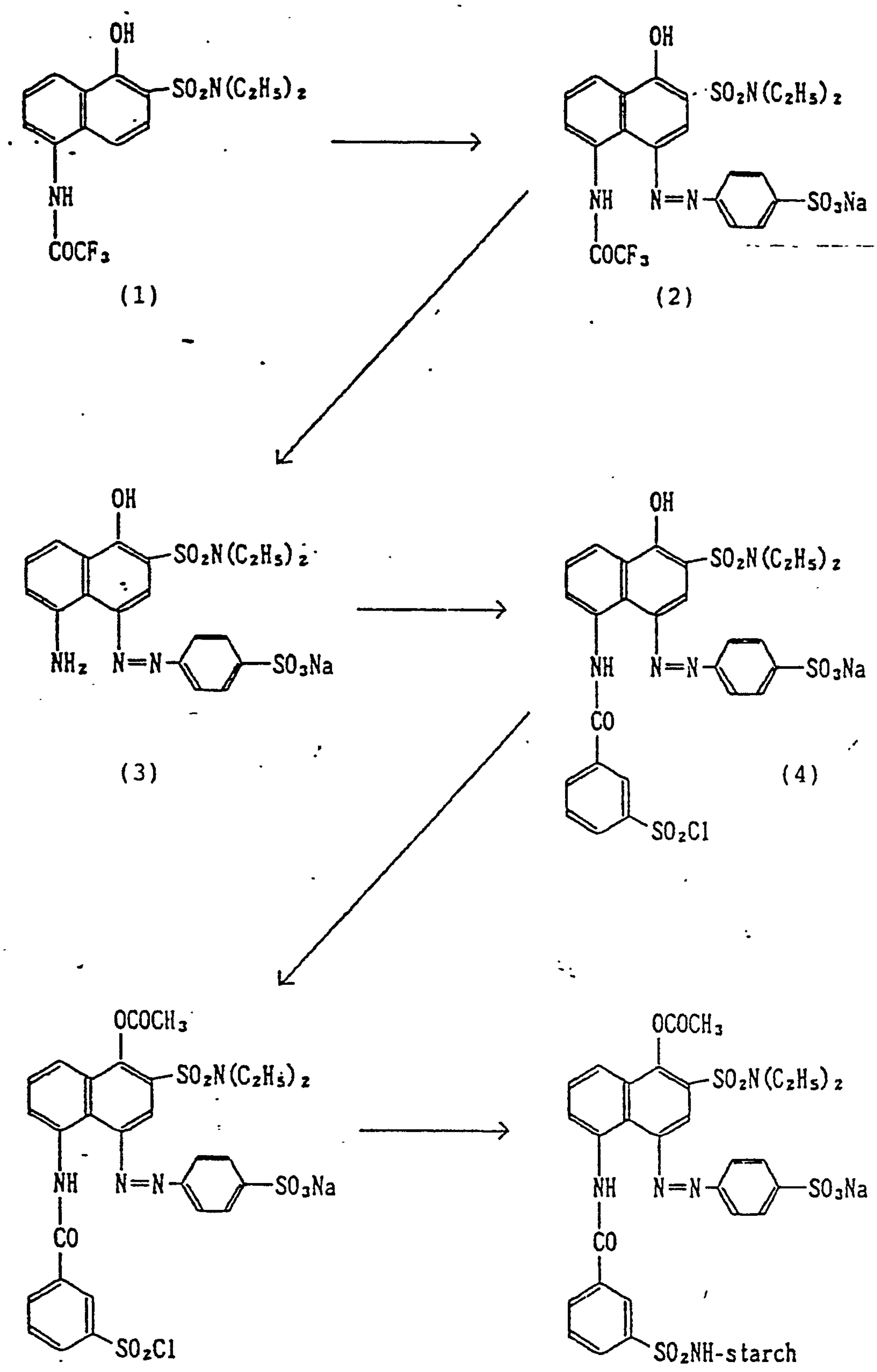

(1) (2) (3) (4) (5) (6)

Production of Compound (2):

Compound (1) (19.5 g, 0.05 mol) was added to a mixture comprising methanol (250 ml), ethylene glycolmonomethylether (25 ml) and soldium acetate (13.5 g). A solution[1]) of diazonium salt derived from sodium sulfanylate (100 ml: 0.0525 mol) was dropwise added to the resulting solution at a temperature not exceeding 10°C. The whole was stirred at 5°C to 10°C for 1 hour and then at room temperature for 1 hour. Red crystals precipitated were taken out by filtration and recrystallized from a mixed solvent of methanolic ethanol (10 v/v%) to obtain compound (2) (16.2 g, 54.4%).

m.p. 245° - 7° $\lambda_{max}$ = 505 nm

1): L.F.Fieser; Org. Synth. Coll. Vol. II, p.35 (1943)

Production of Compound (3):

A crystal of compound (2) (15 g, 8.39 mmol) was added to a solution comprising sodium hydroxide (5 g) and water (104 ml). After stirring for 1.5 hours at room temperature, a saturated sodium chloride solution (500 ml) was added thereto and then acetic acid (10 ml) was dropwise added thereto. A dark red crystal precipitated was taken out by filtration and dried in a desiccator to obtain compound (3) (10.95 g, 87%).

m.p. > 250°

Production of Compound (4):

A solution of compound (3) (500 mg, 1 mmol) in water (10 ml) and 1,1,3,3-tetramethylurea (0.5 ml) was dropwise added to a solution comprising acetonitrile (10 ml) and meta-sulfobenzoic acid dichloride (300 mg, 1.25 mmol). After stirring for 10 minutes at room temperature, the resulting reaction solution was poured into a saturated sodium chloride solution (150 ml). A substance precipitated was taken out by filtration, and this was dissolved in acetone (100 ml) and then dried with sodium sulfate. After the solvent was evaporated out under reduced pressure, compound (4) (270 mg, 38.4%) was obtained.

m.p. > 250°

$\lambda_{max}$ = 510 nm,

Molecular absorbancy index ($\epsilon$ value) = 23000

Production of Compound (5):

Compound (4) (1.0 g, 1.42 mmol) was dissolved in acetone (80 ml) and cooled to 0 to 5°C. Triethylamine (0.8 ml) was gradually and dropwise added to the resulting solution. Next, acetyl chloride (1.2 ml) was added thereto and then the whole was gradually warmed up to room temperature. After stirring for 1 hour at room temperature, this was filtered. Ethyl acetate (300 ml) was added to the resulting filtrate. After washing with an aqeous 3% hydrochloric acid solution (50 ml x 3) and a saturated sodium chloride solution (50 ml x 3), this was dried with magnesium sulfate. The solvent was evaporated out under reduced pressure to obtain compound (5) (550 mg, 51.9%).

m.p. > 250°

Production of Nitroamylopectin:

Amylopectin (derived from potato, by Sigma Co.) (14 g) and p-nitrophenylisocyanate (2.45 g) were added to pyridine (240 ml) and stirred for 20 hours at 100°C. After cooling, acetone (1 liter) was added and the resulting mixture was filtered. The residue obtained was well washed with acetone ( 1 liter) and ethnol (1 liter) and then dried with a vacuum pump to obtain a nitroamylopectin (15 g). By elemental analysis (nitrogen content) it was found that the product contained one substituent per 40 glucose units.

Production of Aminoamylopectin:

The above-prepared nitroamylopectin (13 g) and sodium hydrosulfite (3.9 g) were added to water (520 ml) and stirred for 1 hour at 70 to 75 °C. After cooling, ethanol (500 ml) was gradually added thereto. A block precipitated was isolated by decantation, and this was well washed with an aqueous ethanol/water (50 v/v%). After being dried, an aminoamylopectin (5.36 g) was obtained.

Production of Compound (6):

The animoamylopectin, (820 mg) was added to a mixed solvent comprising dimethylacetamide (DMAC) (16 ml) and water (1 ml) and well stirred. A DMAC solution (2 ml) of the compound (5) (320 mg) was added to the resulting solution whereby an yellow substance precipitated out. The precipitate was taken out by filtration and then well washed with methanolic chloroform (70 v/v%)). After being dried, a temporarily short-shifted amylopectin (corresponding to the above-mentioned compound (6)) (300 mg) was obtained.

Specific examples of the nucleophilic reagents to be used in the present invention for re-coloring the temporarily short-shifted dye moiety-containing diffusible compound to a substance with a long wavelength absorption are shown below.

N-1

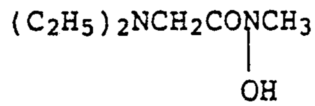

N-2

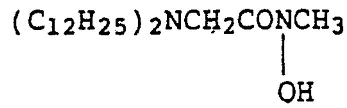

N-3 $(C_{12}H_{25})_2NCH_2CONHOH$

N-4

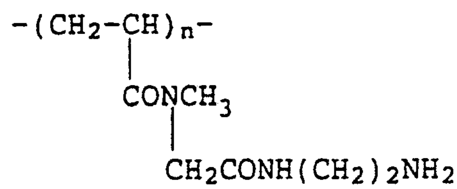

Molecular Weight: 20,000 to 500,000

N-5

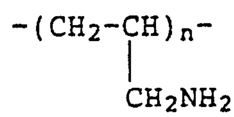

Molecular Weight: 20,000 to 80,000

N-6

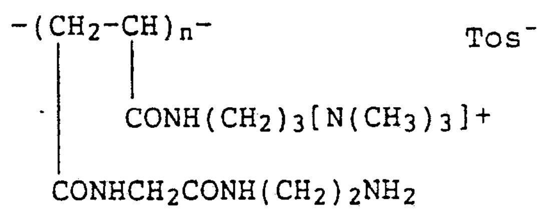

Tos: p-toluenesulfonate anion

Molecular Weight: 20,000 to 500,000

N-7

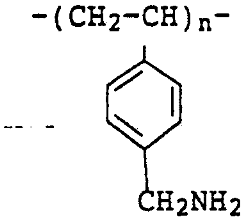

Molecular Weight: 20,000 to 80,000

In the analyzing element of the present invention, the substrate layer may be the uppermost layer of the element or may be between the uppermost layer and the color reaction layer. When the substrate layer is

one containing the non-diffusible substance in a layered continuous phase, the substrate layer may be formed by coating. In this case, since the substrate itself is a polymer substance, the substrate layer may also be formed merely by coating a binder-free substrate-containing liquid composition.

In addition, another method can also be employed by which the non-diffusible substance is dissolved or dispersed in a liquid comprising one or more hydrophilic polymers and the resulting solution or dispersion is coated and dried. As the hydrophilic polymers for use in coating the substrate layer, there may be mentioned, for example, gelatin and derivatives thereof (such as phthalated gelatin), cellulose derivatives (such as carboxymethyl cellulose, hydroxyethyl cellulose), agarose, sodium alginate, polyacrylamide, polymethacrylamide, copolymers of acrylamide or methacrylamide and various vinyl monomers, polyhydroxyethyl methacrylate, polyvinyl alcohol, polyvinyl pyrrolidone, sodium polyacrylate, copolymers of acrylic acid and various vinyl monomers, copolymers of maleic acid and various vinyl monomers, etc. Suitable polymers can be selected from among them, in consideration of the charactersitics of the sample liquid and the substance to be tested, the reaction system, the coating characteristics, etc.

The substrate layer may additionally contain an enzyme activator, a co-enzyme, a surfactant, a pH adjusting reagent, a fine powder, an antioxidant as well as other various inorganic or organic additives, for the purpose of improving the coating property as well as the diffusiveness, reactivity, storage stability and other various properties of the diffusible compound to be formed in the layer.

The substrate layer may be porous. The porous layer may be fibrous or non-fibrous. The layer may be made of anyone of a natural fiber cloth, a synthetic or semi-synthetic fiber cloth, a non-woven fabric, paper, a membrane filter of cellulose acetate, etc. as well as an inorganic or organic grain-bound substance. For example, the fibrous layers described in JP-A-55-164356 (U.S. Patent 4,292,272), 60-222769, etc. as well as the porous layers described in JP-A-49-53888 (U.S. Patent 3,992,158), 58-70163, 61-4959 (European Patent Application 0166365A), 62-116258, 62-138756, 62-138757, 62-138758, etc. are useful. The porous layer may be a liquid-spreading layer (hereinafter often referred to as a "spreading layer") having a so-called metering function of spreading the liquid applied thereto to the area which is almost in proportion to the amount of the said liquid.

For example, the non-diffusible substance is previously applied to paper, cloth or a porous film made of polymers by dipping or coating and the resulting product is adhered to a water-permeable layer, for example, a color reaction layer formed on a support, using the method described in the above-mentioned JP-A-55-164356 (U.S. Patent 4,292,272). Alternatively, a porous layer containing the non-diffusible substance which has previously been applied by dipping or coating is adhered to a porous layer, such as a color reaction layers formed on a support, using the method described in the above-mentioned JP-A-61-4959. As another embodiment, it is also possible to first adhere a porous layer to a water-permeable layer (such as color reaction layer) by the method mentioned above and then to coat the non-diffusible substance-containing composition over the resulting porous layer.

A conventional known method can be utilized for applying the substrate to the porous layer by dipping or coating. For coating, for example, dip coating, doctor coating, hopper coating, curtain coating or the like can properly be selected and used.

The thickness of the substrate layer is not specifically limited. When the layer is formed in the form of a coated layer, the thickness thereof is preferably from 1 µm to 50 µm or so, more preferably from 2 µm to 30 µm or so. When this is formed by any other method than coating, for example, by laminating, the thickness may broadly vary in the range, generally, of from 30 µm to 800 µm or so.

As the color reaction layer in the analyzing element of the present invention there can be used a substantially uniform layer having a hydrophilic polymer as a binder as well as other porous layers described, for example, in JP-A-58-70163 (U.S. Patent 4,486,537), 61-4959, 62-116258, 62-138756, 62-138757, 62-138758, etc. As the hydrophilic polymer there can be used, for example, gelatin and derivatives thereof (such as phthalated gelatin), cellulose derivatives (such as hydroxyethyl cellulose), agarose, sodium alginate, acrylamide polymers, methacrylamide polymers, copolymers of acrylamide or meth acrylamide and various vinyl monomers, polyhydroxyethyl methacrylate, polyvinyl, alcohol, polyvinyl pyrrolidone, sodium polyacrylate, copolymers of maleic acid and various vinyl monomers, copolymers of acrylic acid and various vinyl monomers, etc.

A known method can be utilized for applying a recoloring reagent to a porous layer by dipping or coating. For coating, for example, dip coating, doctor coating, hopper coating, curtain coating, etc. can be utilized.

The thickness of the color reaction layer is preferably from 0.1 to 50 µm, more preferably from 0.2 to 20 µm, and the amount of the color restoration agent in the analyzing element is preferably from 0.1 to 30 $g/m^2$, more preferably from 0.2 to 10 $g/m^2$.

The color reaction layer may further contain, in addition to the hydrophilic binder, a surfactant, a pH

adjusting reagent, a fine powder, an antioxidant as well as other various organic or inorganic additives, for the purpose of improving the coating property and the diffusiveness, reactivity, storability and other various properties of the diffusible compound accepted by the layer. Examples of buffers which can be incorporated into the color reaction layer include carbonates, borates, phosphates as well as Good's buffers described in Biochemistry, Vol. 5, No. 2, pages 467 to 477 (1966), etc.

The material used for preparation of the separating layer is hydrophilic, and can be selected from the materials which are recited hereinabove as hydrophilic polymers used in the substrate layer or the color reaction layer. Additives for improving the coating property and the mobility of the diffusible compound may also be added to this layer. The thickness of the separating layer is perferably from 1 to 30 µm, more preferably from 2 to 20 µm.

In the case of measuring a trace amount of a substance in a humor (hereinafter referred to as "ligand") by immunoassay, an enzyme immunoassay is an effective means for measurement. The analyzing element of the present invention is useful for measuring the activity of an enzyme-labeled substance (or the amount thereof present) in an enzyme immunoassay, and thus the amount of the ligand present in a test sample can be measured by the use of the analyzing element of the present invention.

The enzyme-labeled substance may be bound to the ligand or a derivative thereof, or may be bound to an antibody specific to the ligand. The ligand derivatives mean derivatives formed by introducing an optionally substituted amino, carboxyl or thio group into the ligand. For example, 17-α-hydroxyprogestrone-4-carboxyethylthioether is a derivative of 17-α-hydroxyprogestrone. Examples of ligand derivatives are described in E. Ishikawa, Enzyme Immunoassy, 2nd Ed. (published by Igaku Shoin, Japan, 1982).

For preparing an enzyme-labeled antigen or antibody is used, various enzymes can be selected for the labeling. The enzymes are selected in consideration of the possibility of obtaining a sufficiently high enzyme activity, the stability of the enzymes, the influence of the bond-formation (between enzyme and antibody or antigen) on the enzymatic activitiy, etc. For example, enzyme can be selected from those described in Table 1 and Table 2 in JP-A-56-500901 (U.S. Patent 4,287,300). Typically, there may be mentioned β-D-galactosidase, alkaline phosphatase (ALP), α-amylase, β-amylase, glucoamylase, cellulase, dextranase, etc.

As to the method of binding an enzyme to an antigen or antibody as well as the enzyme-labeled antigen for use in the present invention, reference may be made to the disclosures of E. Ishikawa et al., Enzyme Immunoassay (2nd Ed.) (1982); T. Kawai, Compendium of Clinical Examination Technique, No. 4, Immune Serum Examination, pages 97 to 102 (published by Igaku Shoin, Japan, 1977); Biochem. Biophys. Res. Commun., 74, 538 (1977); Clinica Chimica Acta, 83, 161 (1978), etc. As examples of labeled antigen and antibody, there are α-amylase-bound human immunoglubulin G (IgG), ALP-bonded IgG, cellulase-labeled α-fetoprotein, etc.

When the analyzing element of the present invention is utilized for an enzyme immunoassay, one preferred embodiment is as follows:

A sample which is expected to contain a ligand, a ligand or derivative thereof bound to an enzyme (labeled ligand) and an antibody specific to the ligand are previously reacted in an aqueous medium, and then a small portion of the resulting reaction solution is spotted to the analyzing element of the present invention. This process may further include a step of separating the labeled ligand bound or not bound to the antibody. The method for the separation is described, for example, in E. Ishikawa, et al., Enzyme Immunoassay (published by Igaku Shoin, 1982).

As another embodiment, a sample which is expected to contain a ligand is previously reacted with an antibody specific to the ligand or a derivative thereof bound to an enzyme (labeled antibody) in an aqueous medium, and thereafter a small lportion of the resulting reaction solution is spotted to the analyzing element of the present invention. The process may also include a step of separating the labeled antibody bound or not bound to the ligand.

As still another preferred embodiment, all or a part of the above-mentioned labeled ligand and antibody to the ligand or labeled antibody may be incorporated into an appropriate layer of the analyzing element of the present invention. Alternatively, an additional layer containing the same may be provided over the analyzing element of the present invention just before use thereof for analysis.

The present invention can be applied to various known dry-type analyzing elements. The element may have a multi-layered constitution comprising a porous layer, a reagent layer (i.e., substrate layer and color reaction layer) and additionally a support, a spreading layer, a detecting layer, a light-shielding layer, an adhesive layer, a filter layer (for filtering obstructive substances such as blood corpuscles), a water-absorbing layer, a subbing layer and other various layers. Examples of such analyzing elements are illustrated, for example, in U.S. Patents 3,992,158 and 4,042,335 and JP-A-55-164356 (U.S. Patent 4,292,272).

When a light-transimitting and water-impermeable support is used, the practical constitution of the dry-type analyzing element of the present invention includes the following embodiment:

(1) A color reaction layer and a substrate-containing spreading layer are laminated in order on a support.

(2) A detecting layer, a color reaction layer and a substrate-containing spreading layer are laminated in this order on a support.

(3) A detecting layer, light-reflective layer, a color reaction layer and a substrate-containing spreading layer are laminated in this order on a support.

(4) A color reaction layer, a substrate layer and a spreading layer are laminated in this order on a support.

(5) A detecting layer, a color reaction layer, a substrate layer and a spreading layer are laminated in this order on a support.

(6) A detecting layer, light-reflective layer, a color reaction layer and a substrate layer and a spreading layer are laminated in this order on a support.

The color reaction layer may comprise plural layers. A water-absorbing layer may be provided between the color reaction layer or the detecting layer and the support. In the above-mentioned embodiments (4) and (6), a light-reflective layer may be provided between the substrate layer and the spreading layer. A filter layer may be provided between the substrate layer and the spreading layer or the color reaction layer. A filter layer may also be provided between the light-reflective layer and the spreading layer.

The detecting layer generally means a layer in which the dye or the like as formed in the presence of a component to be tested diffuses and is optically detected through the light-transmissible support, and this may be made of a hydrophilic polymer. This may contain a mordant agent, for example, a cationic polymer to an anionic dye. The water-absorbing layer substantially does not permit the dye formed in the presence of a component to be tested to diffuse therein, and this may be made of a swellable hydrophilic polymer.

As a meterial for the light-transmissible and water-impermeable support, polyethylene terephthalate is preferred. In general, a subbing layer is coated on the support or hydrophilicity is imparted to the support so that the said hydrophilic layer may strongly be adhered to the support.

If the spreading layer is porous, this layer is desirably has a fluid-metering function. The fluid-metering function of the spreading layer means that the liquid sample as spotwise applied to the surface of the layer may be spread over the surface thereof almost in a constant volume per unit area in the direction of the plane, without substantially localizing the components contained in the sample.

As a material for making the spreading layer or other porous layers, there are filter paper, unwoven fabric, woven fabrics (e.g., plane weave fabric), knitted fabrics (e.g., tricot fabric , glass fibers, filter paper, etc. Preferred materials are woven fabrics and knitted fabrics. Woven fabrics may be processed by glow-discharge treatment, as described in JP-A-57-66359 (British Patent 2,087,074). The spreading layer may contain the hydrophilic polymer or surfactant described in JP-A-60-222770 (European Patent Application 162,301A), Japanese Patent Application Nos. 61-122875, 61-122876, so as to properly control the area to be spreaded, the spreading speed, etc.

An adhesive layer for adhering and laminating a porous layer may be provided on the color reaction layer, light-reflective layer, filter layer, water-absorbing layer, etc. The adhesive layer desirably is made of a hydrophilic polymer which may be adhesive to a layer when swollen with water, for example, galatin, gelatin derivatives, polyacrylamide, starch, etc.

The light-reflective layer has the function of shielding the color of the liquid sample as spotwise applied to the spreading layer, in particular, the red color of hemoglobin in a whole blood sample, in the reflection photometry of the detectable variation (color change, coloration, etc.) as yielded in the detecting layer or the reagent layer, from the side of the support, and further has the function of a background layer. The light-reflecting layer is preferred to be a water-permeable layer containing light-reflective fine grains of titanium oxide, barium sulfate or the like dispersed in a hydrophilic polymer used as a binder. The binder is preferably selected from gelatin, galatin derivatives and polyacrylamide. In the analyzing element of the present invention, light-reflective grains such as titanium oxide or the like can be incorporated into at least one of the spreading layer, substrate layer, color reaction layer, detecting layer, etc., in place of providing the light-reflective layer or in addition thereto.

The following referential example and examples are intended to illustrate the present invention in greater detail but not to limit it in any way.

Unless otherwise specified all percents, ratios, etc. are by weight.

## REFERENTIAL EXAMPLE

### (1) Preparation of Color Reaction Test Elements:

A liquid composition (A) comprising the components mentioned below was coated over a gelatin-subbed, colorless, transparent and smooth polyethylene terephthalate film (180 µm thick) to form a coat (coloring reagent layer) having a dry thickness of 12 µm and then dried.

Composition (A):

Gelatin 10 g
Distilled water 190 ml
Compound N-5 3,g

An aqueous 5% gelatin solution was overcoated on the reagent layer in a dry thickness of 2 µm and dried to form a timing layer (separating layer).

The reagent layer and the timing layer were wetted with about 30 g/m$^2$ of water and then a tricot fabric made of polyethylene terephthalate spun yarns (36 gauge) was adhered thereto under pressure and dried to form a spreading layer. The thus prepared color reaction test film was cut into a size of 15 × 15 mm and this was fitted to a 24 × 28 mm plastic frame having a window with a diameter of 10 mm. Thus a color reaction test element (1) was prepared.

For comparison, a comparative color reaction test element (2) was also prepared in the same manner as above except that the composition (A) not containing the Compound N-5 was used.

### (2) Color Reaction Test:

10 µl of an aqueous solution of the compound (5) prepared in the aforesaid production example (0.5 mg/ml) was dropped on the spreading layer of the above-prepared color reaction test elements. Using a Fuji Drychem 1000 Analyzer (manufactured by Fuji Photo Film Co.), the variation of the reflective optical density at a wavelength of 510 nm at 37°C was determined. The results are shown in Fig. 4.

It is understood from the results in Fig. 4 that compound (5) was deacylated in the color reaction test element so that the maximum absorption wavelength was shifted to the longer wavelength side.

## EXAMPLE

A liquid composition (A) comprising the components mentioned below was coated over a gelatin-subbed, colorless, transparent and smooth polyethylene terephthalate film (180 µm thick) to form a coat (reagent layer) having a dry thickness of 12 µm and then dried.

(A)

Gelatin 10 g
Distilled water 190 ml
Compound N-5 3 g

An aqueous 5% gelatin solution was overcoated on the reagent layer in a dry thickness of 2 µm and then dried (timing layer).

The reagent layer and the timing layer were wetted with about 30 g/m$^2$ of water and then a tricot fabric made of polyethylene terephthalate spun yarns (36 gauge) was adhered thereto under pressure and dried to form a spreading layer.

A liquid composition (B) having the components mentioned below was overcoated on the spreading

layer in an amount of 120 ml/m$^2$ and then dried.

(B)

Compound (6) prepared, in Production Example 3 g
Nonylphenoxy-polyethyoxyethanol(*) 190 ml
Distilled Water 100 ml
(*) Number of the ethoxy group = 10

The thus prepared color reaction test film was cut into a size of 15 x 15 mm and this was fitted to a 24 x 28 mm plastic frame having a window with a diameter of 10 mm. Thus a dry-type analyzing element (a) for amylase activity measurement was obtained.

For comparison, a comparative analyzing element (b) was also prepared in the same manner as above except that composition (A) not containing Compound N-5 was used.

10 µl of a buffer solution (tris(hydroxymethyl)amino methane-HCl 50 mM, pH 7) containing a human salivary amylase (manufactured by Sigma Co.) in an amount of 0, 250, 500 or 1000 International Units/ml was spotted on the analyzing elements and, after being reacted for 6 minutes at 37° C, the reflective optical density at a wavelength of 510 nm was measured with an optical system comprising a Fuji Drychem 1000 Analyzer (manufactured by Fuji Photo Film Co.). The results obtained are shown in Table 1 below.

As is obvious from the results in Table 1, the amylase activity-analyzing element of the present invention yielded a distinct coloration (re-coloration) to the amylase activity of from 250 to 1000 units/ml.

Table 1

| Amylase Activity* | Element (1) of the Invention | Comparative Element (2) |
|---|---|---|
| 0 | 0.42 | 0.42 |
| 250 | 0.53 | 0.41 |
| 500 | 0.65 | 0.43 |
| 1000 | 0.87 | 0.40 |

* International Unit/ml

While the invention has been described in detail and with reference to specific embodiments thereof, it will be apparent to one skilled in the art that various changes and modifications can be made therein without departing from the spirit and scope thereof.

## Claims

1. A multi-layer dry-type chemical analyzing element capable of analysis of a particular component in an aqueous liquid sample, which comprises at least two water-permeable layers and which is wherein one of the at least two water-permeable layers is a substrate layer containing a substrate of an enzyme and the other water-permeable layer is a color reaction layer, the substrate is chemically bound with a dye moiety with a temporarily hypsochromically shifted spectral absorption maximum to form a non-diffusible substance which reacts with a substance having an enzyme activity to form a diffusible substance containing a dye moiety with a temporarily hypsochromically shifted spectral absorption maximum in the molecule, and the color reaction layer contains a reactive substance which reacts with the diffusible substance to thereby lengthen the wavelength of the maximum spectral absorption of the temporarily shifted dye moiety.

2. A multi-layer dry-type chemical analyzing element as in claim 1, wherein the non-diffusive substance forms the diffusible substance by enzyme activity of the component to be determined or analyzed in the sample.

3. A multi-layer dry-type chemical analyzing element as in claim 1, wherein the non-diffusible substance forms the diffusible substance by enzymatic activity of an enzyme-carrying substance to be formed or decreased in accordance with the amount of the component to be determined or analyzed in the sample.

4. A multi-layer dry-type chemical analyzing element as in claim 1, wherein the color reaction layer and the substrate layer are laminated in this order on a light-transmissible and water-impermeable support.

5. A multi-layer dry-type chemical analyzing element as in claim 1, wherein a separating layer is provided between the color reaction layer and the substrate layer.

6. A multi-layer dry-type chemical analyzing element as in claim 1, wherein a spreading layer is provided as the uppermost layer.

7. A multi-layer dry-type chemical analyzing element as in claim 1, wherein the wavelength of the maximum spectral absorption of the diffusible compound is shifted by reacting with the reactive substance in the width of from 30 nm to 150 nm.

8. A multi-layer dry-type chemical analyzing element as in claim 1, wherein the maximum absorption wavelength of the dye moiety after lengthening thereof is from 450 to 650 nm.

9. A multi-layer dry-type chemical analyzing element as in claim 1, wherein the wavelength of the maximum spectral of the dye moiety in the non-diffusible substance is shortened by protecting the auxochrome of the dye moiety.

10. A multi-layer dry-type chemical analyzing element as in claim 9, wherein the dye moiety has an auxochrome having a pKa value of not more than 6.0.

11. A multi-layer dry-type chemical analyzing element as in claim 1, wherein the reactive substance is a nucleophilic agent.

12. A multi-layer dry-type chemical analyzing element as in claim 1, wherein the reactive substance is at least one compound selected from the group consisting of primary amines, secondary amines, thiols, alkoxides, hydroxamic acids, oximes, hydrazines, and hydroxylamines.

13. A multi-layer dry-type chemical analyzing element as in claim 1, wherein the substrate is naturally occurring polymeric polysaccharide.

Fig. 1

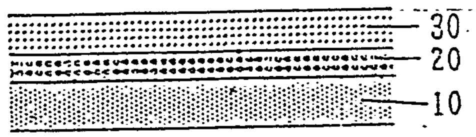


Fig. 2

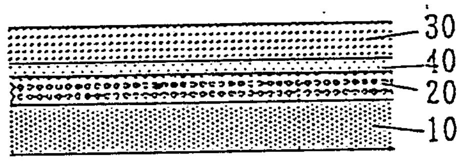


Fig. 3

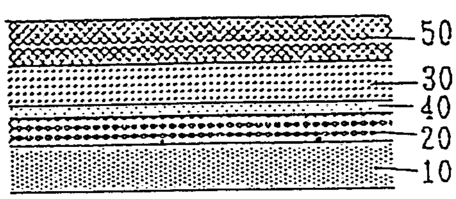

Fig. 4

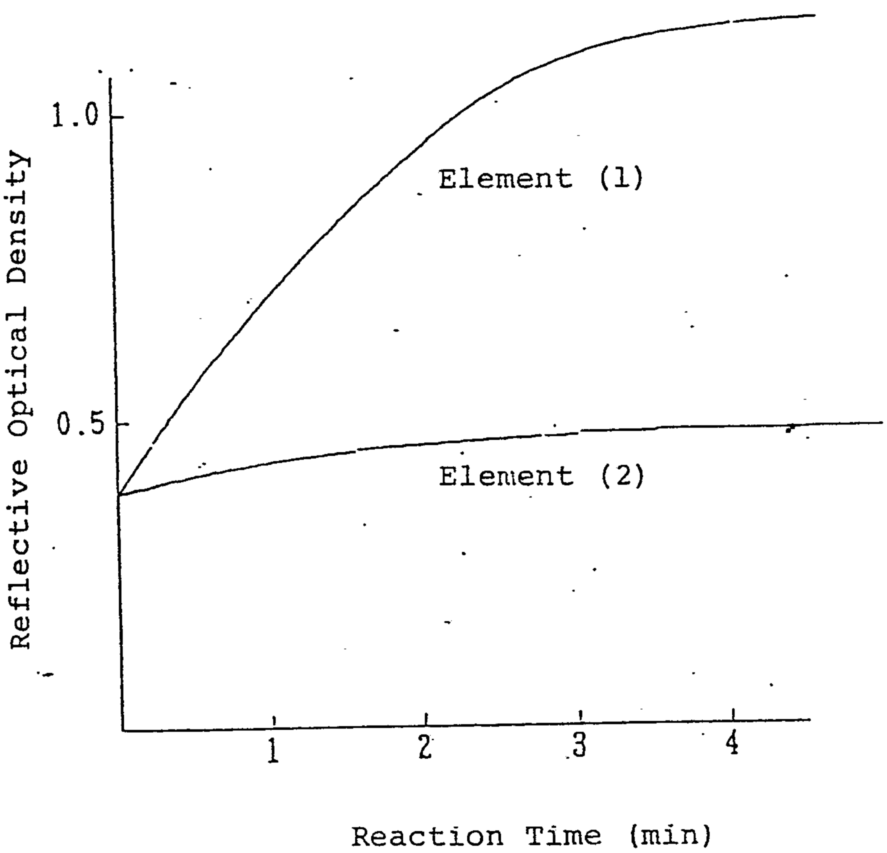
1.0
0.5
Reflective Optical Density
Element (1)
Element (2)
1
2
3
4
Reaction Time (min)